(19) Europäisches Patentamt
European Patent Office
Office européen des brevets

(11) **EP 1 935 333 B1**

(12) **EUROPEAN PATENT SPECIFICATION**

(45) Date of publication and mention
of the grant of the patent:
**18.05.2016 Bulletin 2016/20**

(51) Int Cl.:
***A61B 5/0205*** *(2006.01)*  ***A61B 5/113*** *(2006.01)*

(21) Application number: **07121959.6**

(22) Date of filing: **30.11.2007**

(54) **Non-contact apparatus for monitoring cardiopulmonary activity signals and method for the same**

Kontaktlose Vorrichtung zur Überwachung der Herz-Lungen-Lebenszeichen und Verfahren dafür

Appareil sans contact pour surveiller des signaux cardio-pulmonaires et son procédé

(84) Designated Contracting States:
**DE FR GB**

(30) Priority: **22.12.2006 CN 200610170013**

(43) Date of publication of application:
**25.06.2008 Bulletin 2008/26**

(73) Proprietor: **Industrial Technology Research Institute**
**310 Judung Township, Hsinchu County**
**Taiwan (CN)**

(72) Inventors:
• **Tao, Teh Ho**
  **Hsinchu City (TW)**
• **Hu, Shih Jen**
  **730, Sinying City, Tainan County (TW)**
• **Kuo, Su Chen**
  **363, Gongguan Township, Miaoli County (TW)**
• **Peng, Jia Hung**
  **300, Hsinchu City (TW)**

(74) Representative: **2K Patentanwälte Blasberg**
**Kewitz & Reichel**
**Partnerschaft mbB**
**Schumannstrasse 27**
**60325 Frankfurt am Main (DE)**

(56) References cited:
WO-A-94/20021     WO-A-2005/092190
WO-A-2007/143535    GB-A- 2 349 759

## Description

## BACKGROUND OF THE INVENTION

The present invention relates to a technology for monitoring cardiopulmonary activity signals, and more particularly, to a non-contact apparatus and method for monitoring cardiopulmonary activity signals

Breathing disorders commonly occur in premature infants, which manifest themselves in symptoms such as apnea or intermittent breathing etc., and can further develop into two types of complications: one of which occurs with apnea of premature birth infants, also known as apnea of prematurity (AOP); the other is sudden infant death syndrome (SIDS) or apparent life threatening event (ALTE). If AOP is not properly diagnosed and treated, premature infants diagnosed with this type of symptom will be highly susceptible to SIDS and are accident-prone at home. Cessation of breathing might lead to hemodynamic chain reactions such as slowdown of the heartbeat rate, and even lowered blood pressure. These changes in hemodynamics can induce temporary loss of blood and oxygen to critical organs, especially to the brain cells and may even cause permanent damage to them. In clinical trials, premature infants often become stable in all other aspects and could be discharged from the hospital, if it were not for this type of irregular apnea symptom.

Infant monitors currently in clinical use are based on traditional bedside monitors of patients' physical condition and use three electrodes attached to the skin of the infant's chest, acquiring the signals from the changes in the chest impedance by breathing and heartbeat signals; these two signals are then converted into the separate breathing frequency and heartbeat rate by the filter. A major drawback of this type of monitoring technology is that prolonged contact with the sensor plates will cause skin redness, sensitivity, or deterioration; furthermore, monitors used clinically are often expensive and therefore not suitable for home caretaking purposes.

To solve the above-mentioned problem, US 4,738,264 disclosed a vibration measurement apparatus that is separated from the patient and is disposed on the bed. The main principle behind the apparatus is that tiny vibrations of the body surfaces from normal breathing and heartbeat are transferred to the measurement apparatus through the bed, and then the apparatus converts the signals into a comprehensive energy index to represent the infant's breathing and heartbeat conditions. The drawback of this technique is that it cannot accurately distinguish and measure the infant's breathing frequency and heartbeat rate, and therefore cannot fulfill the clinical requirements for detecting the AOP alert threshold (i.e. breathing frequency is less than 20/minute of breathing ceases for over 15 seconds, and heartbeat rate is less than 80/minute).

In addition, US 5,964,720 disclosed a distributed vibration measurement system that uses the piezoelectric crystals as the vibration sensors and uses strips of conducting film as the base of the sensors. These conductive strips can be embedded in the bed mattresses, seat backs, or cushions to detect tiny vibrations of the body surface resulting from normal breathing and the heartbeat of the patient. The major drawback of this technique is that the heart and chest vibration signals captured by the sensors tend to be subject to interference from vibration noise from the surroundings that travel through the human body and bed or seat to the sensor.

WO 2005/092190 A1 discloses a non-contact apparatus for monitoring cardiopulmonary activity signals, wherein signals in the frequency range between 300 GHz and 30 THz are scattered from a body portion of a human being. A detection setup monitors the coincidence between a reference pulse and the phase-shifted reflected pulse, measures the delay time from a phase-shift signal and converts this into further signals. The delay time between the phase shifted reflected pulse and the reference pulse is swept over a certain delay time range using a delay circuit. This detection setup necessarily requires a delay circuit. In addition generation and detection of radiation in the THz-frequency range requires a complex setup.

WO 94/20021 discloses an ultrasonic monitor for monitoring movement, wherein ultrasound pulses are used.

GB 2 349 759 discloses a device for monitoring the heartbeat of a living body, which comprises a radar which directs a continuous or pulsed beam of microwave radiation towards a body to be monitored. A phase-shift signal reflected from the human body is filtered using a controllable filter, which is adjusted to pass a frequency spectrum at the frequency of either the heartbeat or breathing rate. For this purpose, the signal of a spectrum analyser is A/D-converted and further processed using a micro-processor.

WO 2007/143535 A2, which is prior art according to Art. 54(3) of the EPIC, discloses a non-contact apparatus and method for monitoring the motion, breathing, heart rate and sleep status of subjects.

## SUMMARY OF THE INVENTION

It is an object of the present invention to provide an enhanced non-contact apparatus and method for reliably monitoring cardiopulmonary activity signals using a simple detection setup.

This problem is solved by a non-contact apparatus for monitoring cardiopulmonary activity signals and by a method according to claim 1 and 6 respectively. Further advantageous embodiments are the subject-matter of the dependent claims.

## BRIEF DESCRIPTION OF THE DRAWINGS

The objectives and advantages of the present invention will become apparent upon reading the following description and upon reference to the accompanying drawings in which:

FIG. 1 illustrates the functional block diagram of a non-contact apparatus for monitoring cardiopulmonary activity signals according to one embodiment of the present invention;

FIG. 2 illustrates the system architecture of the non-contact apparatus according to one embodiment of the present invention;

FIG. 3 illustrates the functional block diagram of the signal-processing module according to one embodiment of the present invention;

FIG. 4 illustrates the output signals of the balance mixer according to one embodiment of the present invention;

FIG. 5 illustrates a method for acquiring cardiopulmonary activity signals according to a first embodiment;

Fig. 6 shows output signals generated using the method of Fig. 5;

FIG. 7 illustrates a method for acquiring cardiopulmonary activity signals according to a second embodiment; and

FIG. 8 illustrates a method for measuring the breathing frequency (heartbeat rate) of a patient using a method according to the present invention the present invention.

## DETAILED DESCRIPTION OF THE INVENTION

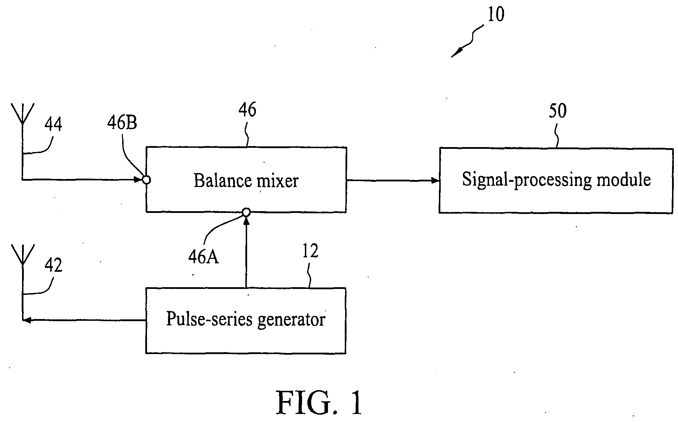

FIG. 1 illustrates the functional block diagram of a non-contact apparatus 10 for monitoring cardiopulmonary activity signals according to one embodiment of the present invention. The non-contact apparatus 10 comprises a pulse-series generator 12 configured to generate a series of high-frequency sinusoidal probing pulses and reference pulses, a transmitting antenna 42 configured to emit the probing pulses to the chest portion to generate a series of scattered pulses from the probing pulses by the scattering of the chest portion, a receiving antenna 44 configured to receive the scattered pulses by the chest portion, a balance mixer 46 having a first input port 46A configured to receive the reference pulses and a second input port 46B electrically connected to the receiving antenna 44 to receive scattered pulses, and a signal-processing module 50 configured to generate the cardiopulmonary activity signals after the scattered pulses and the reference pulses are mixed by the balance mixer 46.

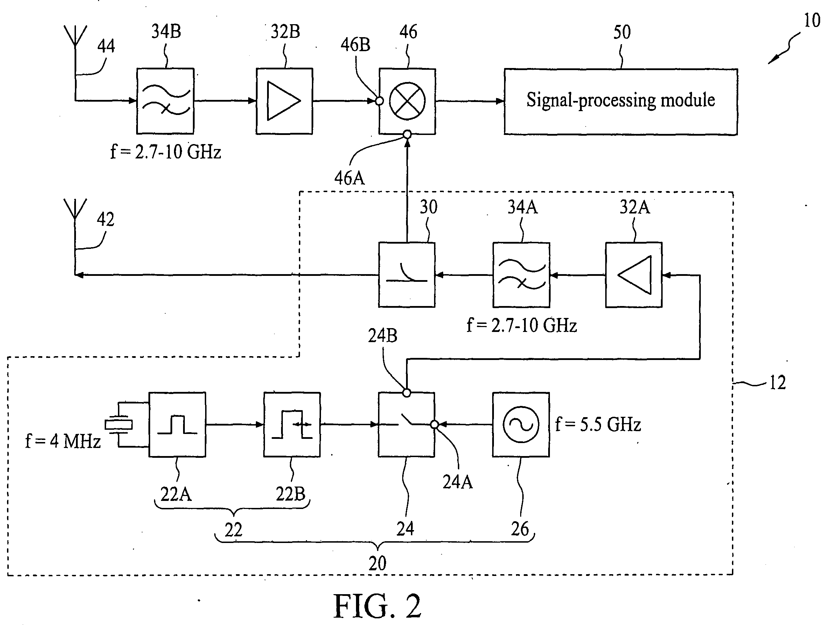

FIG. 2 illustrates the system architecture of the non-contact apparatus 10 according to one embodiment of the present invention. The pulse-series generator 12 comprises a pulse generator 20 configured to generate a series of high-frequency sinusoidal pulses, from which the probing pulses and the reference pulses are generated by the splitter 30 such as a Wilkinson splitter coupled to the high-frequency filter 34A. The pulse generator 20 comprises a sinusoidal-signal generator 26 configured to generate a 5.5GHz continuous sinusoidal signal, a switching-signal generator 22 configured to generate a switching signal, and a switching device 24 having an input port 24A and an output port 24B and configured to turn on according to the switching signal such that the continuous sinusoidal signal can pass through the switching device 22 to form the high-frequency sinusoidal pulses. The switching-signal generator 22 comprises a clock generator 22A configured to generate a clock signal, and a waveform shaper 22B configured to adjust the time interval, 6.0 ns nominal, of the clock signal to generate the switching signal.

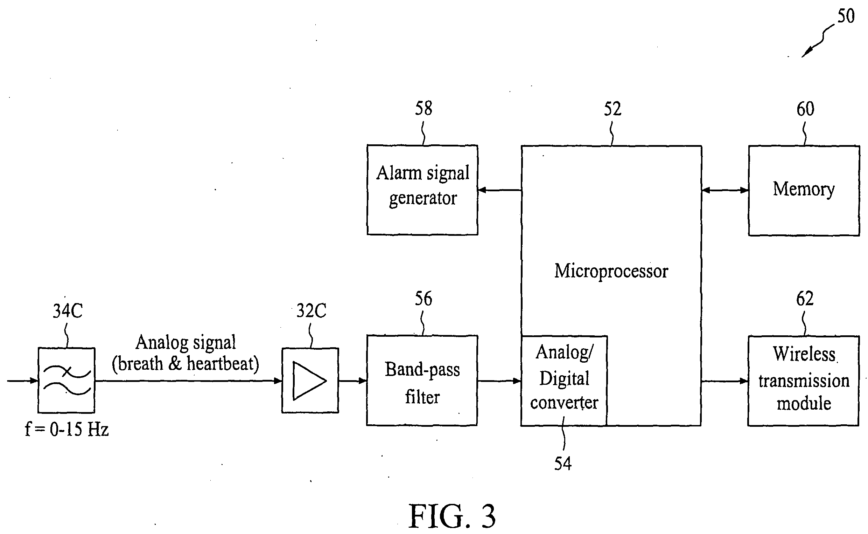

The non-contact apparatus 10 further comprises a first amplifier 32A electrically coupled to the output port of the pulse generator 20, a first high-frequency filter 34A electrically coupled to the first amplifier 32A and the splitter 30, a second high-frequency filter 34B electrically coupled to the receiving antenna 44, and a second low noise amplifier 32B electrically coupled to the second high-frequency filter 34B and the balance mixer 46. FIG. 3 illustrates the functional block diagram of the signal-processing module 50 according to one embodiment of the present invention. The signal-processing module 50 comprises a low-frequency filter 34C electrically coupled to the output port of the balance mixer 46, a third amplifier 32C electrically coupled to the low-frequency filter 34C, a band-pass filter 56 electrically coupled to the third amplifier 32C, a microprocessor 52 with a built-in analog/digital converter 54 electrically coupled to the band-pass filter 56, an alarm signal generator 58, a memory unit 60 configured to store data, and a wireless transmission module 62.

The signal-processing module 50 serves to output the analog signal representing breathing or heartbeat, and the analog/digital converter 54 then converts the analog signal into a digital signal. The breathing or heartbeat signals can be extracted from the digital signal by the firmware of the microprocessor 52, and the breathing frequency or heartbeat rate can be calculated with a certain signal-processing algorithm. The alarm signal generator 58 is configured to send an alarm signal when there is an anomaly, and the processed signals can be sent through the wireless transmission module 62 to the data server for further statistical analysis, printing, and storage.

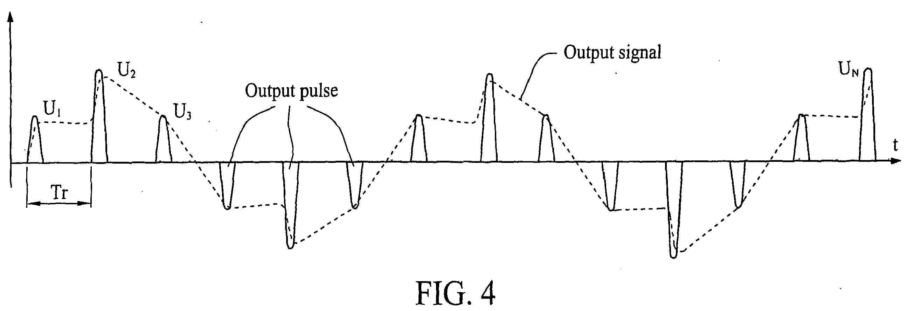

FIG. 4 illustrates the output signals of the balance mixer 46 according to one embodiment of the present invention. The high-frequency sinusoidal pulses are filtered by the first high-frequency filter 34A and then split into two parts by the splitter 30; one is the probing pulses transmitted to the chest portion by the transmitting antenna 42 and the other is the reference pulses fed into the first input port 46A of the balance mixer 46. The transmitted high-frequency sinusoidal probing pulses are then scattered by the chest portion of the subject to generate the scattered pulses, which is then received by the receiving antenna 44. Subsequently, the scattered pulses are mixed with the high-frequency sinusoidal reference pulses by the balance mixer 46. The output of the balance mixer 46 is a series of high-frequency pulses, with the polarity and amplitude of each pulse corresponding to the phase difference of the scattered pulses and the reference pulses. The low-frequency filter 34C will capture the envelop (the dotted lines in FIG. 4) of the high-frequency pulses to obtain the breathing and heartbeat signals of the patient, while the third amplifier 32C will magnify the amplitude of the signal and the band-pass filter 56 further removes baseline drift and high frequency noises.

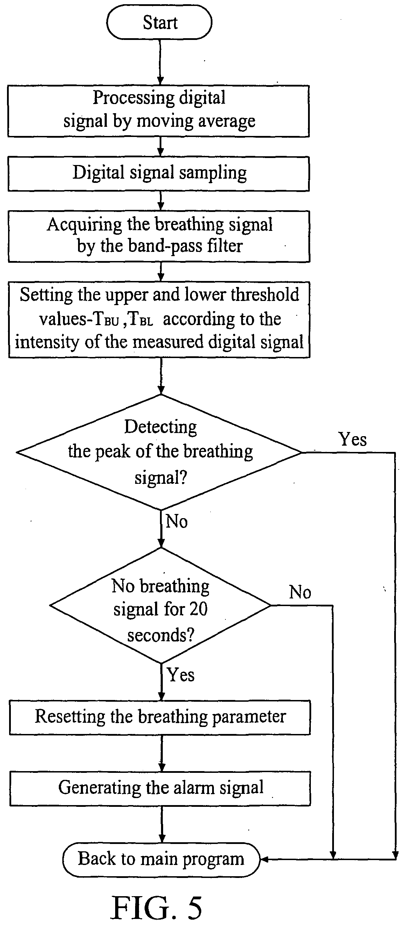

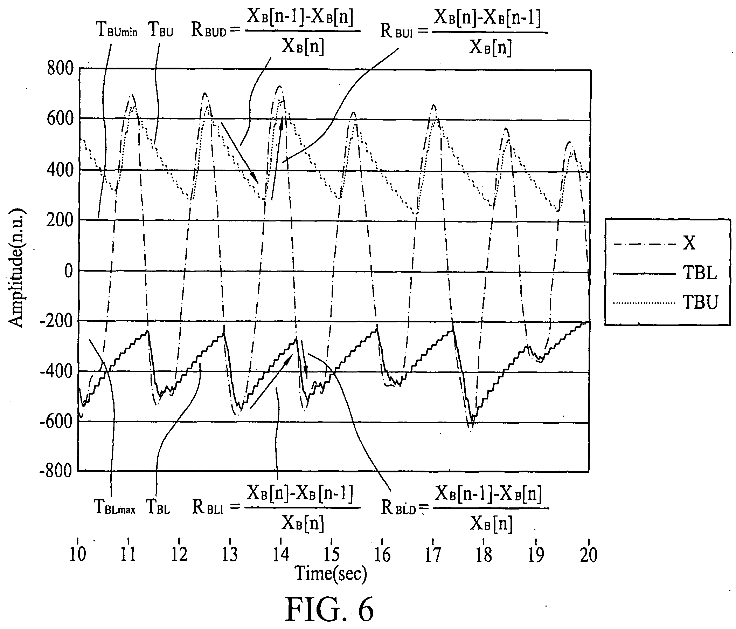

FIG. 5 illustrates a method for processing the digital signal after it is generated by the mixer 46 according to a first embodiment. The moving average method is used to reduce the high frequency noise. A band-pass filter with -3dB bandwidth from 0.1Hz to 0.5Hz is employed to reduce the noise outside the range of subject's normal range of breathing. To set-up the threshold values, the minimum of the upper threshold value ($T_{Bumin}$) and the maximum of the lower threshold value ($T_{BLmax}$), are first defined as two times of the magnitude of the background noise. For example, if the background noise is between $-100 \sim 100$, then the $T_{BUmin}$ is 200 and $T_{BLmax}$ is -200. Then the upper threshold value ($T_{BU}$) and the lower threshold ($T_{BL}$) are adjusted as shown in FIG.6. If the signal value $X_B$ [n] is larger than $T_{BU}$[n], the value of $T_{BU}$ [n+1] is increased with ratio $R_{BUI}$.

$$T_{BU}\ [n+1] = (1+ R_{BUI})^*\ T_{BU}\ [n]$$

$$\text{Where } R_{BUI} = (X_B[n] - X_B[n-1])/X_B[n]$$

If the signal value $X_B$[n] is smaller than $T_{BU}$ [n], the upper threshold value $T_{BU}$[n+1] is then reduced with ratio $R_{BUD}$.

$$T_{BU}\ [n+1] = (1-R_{BUD})^*T_{BU}\ [n]$$

$$\text{Where } R_{BUD} = (X_B[n-1] - X_B[n])/X_B[n]$$

Similarly, if the signal value is less than the lower threshold, the lower threshold is reduced with ratio $R_{BLD}$.

$$T_{BL}\ [n+1] = (1- R_{BLD})^*\ T_{BL}\ [n]$$

$$\text{Where } R_{BLD} = (X_B[n-1] - X_B[n])/X_B[n]$$

If the signal value is higher than the lower threshold, the lower threshold is then increased with ratio $R_{BLI}$.

$$T_{BL}\ [n+1] = (1+R_{BLI})^*T_{BL}[n]$$

$$\text{Where } R_{BLI} = (X_B[n] - X_B[n-1])/X_B[n]$$

Subsequently, the intensity of the measured digital signal is compared with thresholds to check whether a signal peak representing the breathing activity is detected. If the signal is larger than $T_{BU}$, the "peak searching procedure" begins to record the location of current maximum value. The location would be continuously updated until the signal lower than $T_{BL}$. If there is no digital signal with intensity greater than $T_{BU}$ for a predetermined time (for example, 20 seconds), an alarm signal will be generated and all breathing parameters will be reset to zero to restart the calculation process.

Fig. 6 shows output signals generated using the method described with reference to Fig. 5 above.

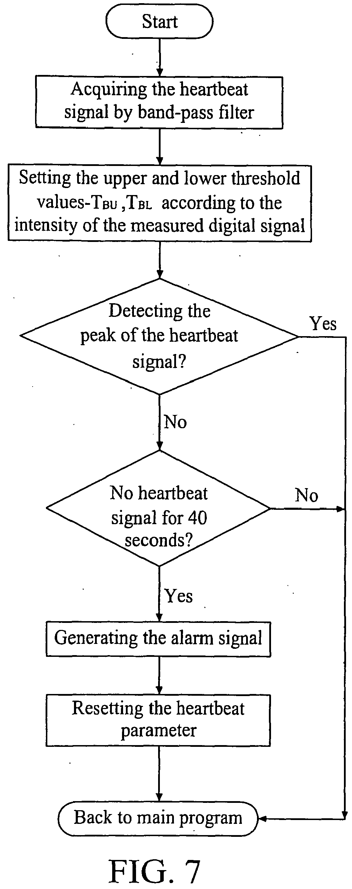

FIG.7 illustrates a method for acquiring cardiopulmonary activity signals according to a second embodiment, in which an alarm signal will be sent when the heartbeat is irregular. The digital signals captured by the non-contact apparatus 10 are heartbeat signals, while the alarm signal represents the cessation of the heartbeat. The microprocessor 52 of the signal-processing module 50 is configured to filter out the heartbeat signal through a band-pass filter with -3dB bandwidth from 0.7 Hz to 2.5 Hz that is implemented in the software, and then set thresholds according to the empirical value or the intensity of the measured digital signal. The "peak searching procedure" is the same as that in breathing signal detection.

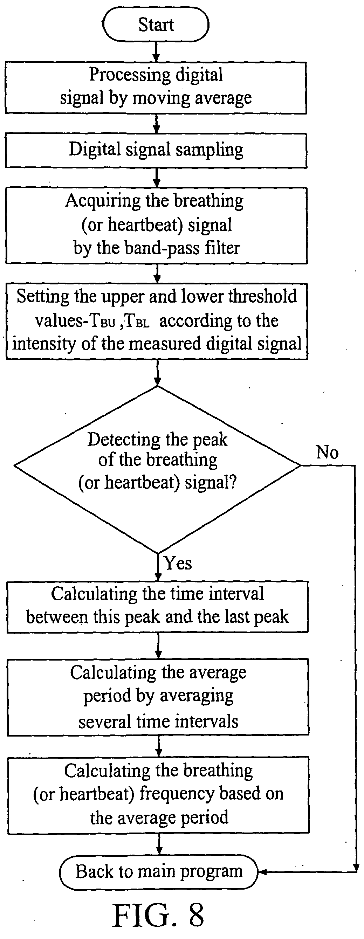

FIG. 8 illustrates a method for measuring the breathing frequency (heartbeat rate) of a patient using a method according to the present invention. The digital signal processing is the same as previous procedure and then peak detecting is performed to record location of each peak. When the peak of the breathing (heartbeat) signals is detected, the time interval between the current breathing (heartbeat) peak and the previous breathing (heartbeat) peak is calculated and saved to the memory 60. The time intervals between a plurality signal peaks are averaged to obtain the average interval, and the breathing frequency (heartbeat rate) is calculated by using the average interval. Furthermore, the present invention can selectively capture a portion of the digital signal (for example setting the sampling rate at 1/5) or discard unstable signals during system startup stage after applying the moving average method to the data signal.

After the calculation of the breathing frequency (heartbeat rate) is completed, the breathing frequency (heartbeat rate) is checked to determine whether it falls in a predetermined range. If the calculated breathing frequency (heartbeat rate) is higher than the predetermined range, an alarm signal indicating the breathing frequency (heartbeat rate) is too high is generated; in contrast, if the calculated breathing frequency (heartbeat rate) is lower than the predetermined range, an alarm signal indicating the breathing frequency (heartbeat rate) is too low is generated.

The above-described embodiments of the present invention are intended for illustration purposes only. Numerous alternative embodiments may be devised by those skilled in the art without departing from the scope of the following claims.

## Claims

1. A non-contact apparatus for monitoring cardiopulmonary activity signals, comprising:

   a pulse-series generator (12) configured to generate a series of probing pulses and a series of reference pulses;
   a transmitting antenna (42) configured to transmit the probing pulses to a chest portion, wherein the chest portion scatters the probing pulses to generate a series of scattered pulses;
   a receiving antenna (44) configured to receive the scattered pulses;
   a mixer (46) including a first input port (46A) configured to receive the reference pulses and a second input port (46B) configured to receive the scattered pulses and connected to the receiving antenna via a first high-frequency filter (34B) electrically connected to an output port of the receiving antenna (44) and a low noise first amplifier (32B) electrically connected to the first high-frequency filter (34B) and the mixer (46); and
   a signal-processing module (50) connected to an output port of the mixer (46) and configured to generate the cardiopulmonary activity signals after the scattered pulses and the reference pulses are processed by the mixer (46);
   wherein the pulse-series generator (12) includes:

   a pulse generator (20) configured to generate a series of high-frequency sinusoidal pulses;
   a second amplifier (32A) electrically connected to an output port of the pulse generator (20);
a second high-frequency filter (34A) electrically connected to the second amplifier (32A); and
a splitter (30) electrically connected to the second high-frequency filter (34A) and configured to generate the probing pulses and the reference pulses from the high-frequency sinusoidal pulses, wherein the frequency band of the probing pulses and of the reference pulses is between 2.7 GHz and 10 GHz; and
wherein the signal-processing module (50) comprises:

   a low-frequency filter (34C) electrically connected to an output port of the mixer (46);
   an third amplifier (34C) electrically connected to an output port of the low-frequency filter (34C);
   a band-pass filter (56) electrically connected to the output port of the third amplifier (34C) and configured for removing baseline drift and high frequency noise; and
   a microprocessor having an analog/digital converter (54) configured to convert the cardiopulmonary activity signals into digital signals; wherein

the microprocessor (52) of the signal-processing module (50) is configured to extract breathing or heartbeat signals from the digital signals through a band pass filter implemented in software with -3dB bandwidth from 0.1 Hz to 0.5 Hz or with -3dB bandwidth from 0.7 Hz to 2.5 Hz, respectively, and to calculate therefrom a respective breathing frequency or heartbeat rate.

2. The non-contact apparatus of Claim 1, wherein the pulse generator (20) includes:

   a sinusoidal-signal generator (26) configured to generate a continuous sinusoidal signal;
   a switching-signal generator (22) configured to generate a switching signal; and
   a switching device (24) configured to turn on according to the switching signal such that the continuous sinusoidal signal can pass through the switching device to form the high-frequency sinusoidal pulses.

3. The non-contact apparatus of Claim 2, wherein the switching-signal generator (22) includes:

   a clock generator (22A) configured to generate a clock signal; and

a waveform shaper (22B) configured to adjust a time interval of the clock signal to generate the switching signal.

4. The non-contact apparatus of any of the preceding claims, wherein the signal-processing module (50) further includes an alarm signal generator (58) electrically connected to the microprocessor.

5. The non-contact apparatus of any of the preceding claims, wherein the mixer is a balance mixer (46).

6. A method for acquiring cardiopulmonary activity signals, comprising the steps of:

generating a series of probing pulses and a series of reference pulses by means of a pulse-series generator (12);

transmitting the probing pulses to a chest portion by a transmitting antenna (42) to generate scattered pulses from the probing pulses by the scattering of the probing pulses at the chest portion;

receiving the scattered pulses by a receiving antenna (44);

generating a phase-difference signal between the scattered pulses and the reference pulses by mixing the scattered pulses with the reference pulses using a mixer (46); and

converting the phase-difference signal into a digital signal representing the cardiopulmonary activity by a signal-processing module (50) connected to an output port of the mixer (46), by processing the scattered pulses and the reference pulses by the mixer (46); wherein

the reference pulses are received by a first input port (46A) of the mixer (46) and the scattered pulses are received by a second input port (46B) of the mixer connected to the receiving antenna via a first high-frequency filter (34B) electrically connected to an output port of the receiving antenna (44) and a low noise first amplifier (32B) electrically connected to the first high-frequency filter (34B) and the mixer (46);

a series of high-frequency sinusoidal pulses is generated by the pulse generator (20), amplified by a second amplifier (32A) electrically connected to an output port of the pulse generator (20) and filtered by a second high-frequency filter (34A) and the probing pulses and the reference pulses are generated from the high-frequency sinusoidal pulses by a splitter (30) electrically connected to the second high-frequency filter (34A), wherein the frequency band of the probing pulses and of the reference pulses is between 2.7 GHz and 10 GHz; and

for converting the phase-difference signal into a digital signal representing the cardiopulmonary activity the phase-difference signal is filtered by a low-frequency filter (34C) electrically connected to the output port of the mixer (46), amplified by a third amplifier (34C) electrically connected to an output port of the low-frequency filter (34C) and filtered by a band-pass filter (56) electrically connected to the output port of the amplifier (34C); wherein

the band-pass filter (56) removes baseline drift and high frequency noise, and

the cardiopulmonary activity signals are converted into digital signals by an analog/digital converter (54) of a microprocessor of the signal-processing module (50), wherein

the microprocessor (52) extracts breathing or heartbeat signals from the digital signals through a band pass filter implemented in software with -3dB bandwidth from 0.1 Hz to 0.5 Hz or with -3dB bandwidth from 0.7 Hz to 2.5 Hz, respectively, and calculates therefrom a respective breathing frequency or heartbeat rate.

7. The method of Claim 6, wherein said step of calculating a breathing frequency or heartbeat rate includes :

setting a threshold value;

checking to determine whether the intensity of the digital signal is greater than the threshold value and determining a signal peak of the cardiopulmonary activity if the checking result is positive;

calculating time intervals of a plurality of signal peaks of the cardiopulmonary activity; and

averaging the time intervals to generate the breathing frequency or heartbeat rate.

8. The method of Claim 6 or 7, wherein breathing signals are extracted from the digital signals.

9. The method of any of claims 6 to 8, further comprising the steps of:

checking to determine whether the calculated breathing frequency or heartbeat rate is in a predetermined range: and

generating an alarm signal if said breathing frequency or heartbeat rate is outside the predetermined range.

10. The method of claim 7, wherein the threshold value is set according to the intensity of the digital signal.

**Patentansprüche**

1. Berührungslose Vorrichtung zur Überwachung von Herz-Lungen-Aktivitätssignalen, umfassend:

einen Impulsserien-Generator (12), der ausgelegt ist, um eine Serie von Untersuchungsimpulsen und eine Serie von Referenzimpulsen zu erzeugen;

eine Sendeantenne (42), die ausgelegt ist, um die Untersuchungsimpulse zu einem Brustabschnitt zu senden, wobei der Brustabschnitt die Untersuchungsimpulse streut, um eine Serie von Streuimpulsen zu erzeugen;

eine Empfangsantenne (44), die ausgelegt ist, um die Streuimpulse zu empfangen;

einen Mischer (46) mit einem ersten Eingangsanschluss (46A), der ausgelegt ist, um die Referenzimpulse zu empfangen, und mit einem zweiten Eingangsanschluss (46B), der ausgelegt ist, um die Streuimpulse zu empfangen, und der über ein erstes Hochfrequenzfilter (34B), das mit einem Ausgangsanschluss der Empfangsantenne (44) leitend verbunden ist, und einen ersten rauscharmen Verstärker (32B), der leitend mit dem ersten Hochfrequenzfilter (34B) und dem Mischer (46) verbunden ist, mit der Empfangsantenne verbunden ist; und

ein Signalverarbeitungsmodul (50), das mit einem Ausgangsanschluss des Mischers (46) verbunden ist und ausgelegt ist, um die Herz-Lungen-Aktivitätssignale zu erzeugen, nachdem die Streuimpulse und die Referenzimpulse durch den Mischer (46) verarbeitet wurden;

wobei der Impulsserien-Generator (12) umfasst:

einen Impulsgenerator (20), der ausgelegt ist, um eine Serie von hochfrequenten sinusförmigen Impulsen zu erzeugen;

einen zweiten Verstärker (32A), der leitend mit einem Ausgangsanschluss des Impulsgenerators (20) verbunden ist;

ein zweites Hochfrequenzfilter (34A), das leitend mit dem zweiten Verstärker (32A) verbunden ist; und

einen Verteiler (30), der leitend mit dem zweiten Hochfrequenzfilter (34A) verbunden ist und ausgelegt, um die Untersuchungsimpulse und die Referenzimpulse aus den hochfrequenten sinusförmigen Impulsen zu erzeugen, wobei das Frequenzband der Untersuchungsimpulse und der Referenzimpulse zwischen 2,7 GHz und 10 GHz liegt; und

wobei das Signalverarbeitungsmodul (50) umfasst:

ein Niederfrequenzfilter (34C), das leitend mit einem Ausgangsanschluss des Mischers (46) verbunden ist;

einen dritten Verstärker (34c), der leitend mit einem Ausgangsanschluss des Niederfrequenzfilters (34C) verbunden ist;

ein Bandpassfilter (56), das leitend mit dem Ausgangsanschluss des dritten Verstärkers (34c) verbunden ist und zum Entfernen einer Basisliniendrift und von hochfrequentem Rauschen ausgelegt ist; und

einen Mikroprozessor mit einem Analog-/Digital-Wandler (54), der ausgelegt ist, um die Herz-Lungen-Aktivitätssignale in digitale Signale zu wandeln; wobei

der Mikroprozessor (52) des Signalverarbeitungsmoduls (50) ausgelegt ist, um aus den digitalen Signalen mit Hilfe eines mittels Software realisierten Bandpassfilters mit einer -3dB-Bandbreite von 0,1 Hz bis 0,5 Hz oder mit einer -3dB-Bandbreite von 0,7 Hz bis 2,5 Hz Atmungs- oder Herzschlag-Signale zu extrahieren und daraus eine jeweilige Atemfrequenz oder Herzschlagfrequenz zu berechnen.

2. Berührungslose Vorrichtung nach Anspruch 1, wobei der Impulsgenerator (20) umfasst:

einen Sinussignal-Generator (26), der ausgelegt ist, um ein kontinuierliches sinusförmiges Signal zu erzeugen;

einen Schaltsignal-Generator (22), der ausgelegt ist, um ein Schaltsignal zu erzeugen; und

eine Schalteinrichtung (24), die ausgelegt ist, um entsprechend dem Schaltsignal einzuschalten, sodass das kontinuierliche sinusförmige Signal die Schalteinrichtung durchlaufen kann, um die hochfrequenten sinusförmigen Impulse auszubilden.

3. Berührungslose Vorrichtung nach Anspruch 2, wobei der Schaltsignal-Generator (22) umfasst:

einen Taktsignal-Generator (22a), der ausgelegt ist, um ein Taktsignal zu erzeugen; und einen Signalformer (22B), der ausgelegt ist, um ein Zeitintervall des Taktsignals einzustellen, um die Schaltsignale zu erzeugen.

4. Berührungslose Vorrichtung nach einem der vorangehenden Ansprüche, wobei das Signalverarbeitungsmodul (50) weiterhin einen Alarmsignal-Generator (58) umfasst, der leitend mit dem Mikroprozessor verbunden ist.

5. Berührungslose Vorrichtung nach einem der vorhergehenden Ansprüche, wobei der Mischer ein Gegentakt-Mischer (46) ist.

**6.** Verfahren zur Erfassung von Herz-Lungen-Aktivitätssignalen, mit den folgenden Schritten:

Erzeugen einer Serie von Untersuchungsimpulsen und einer Serie von Referenzimpulsen mit Hilfe eines Impulsserien-Generators (12); Senden der Untersuchungsimpulse zu einem Brustabschnitt mittels einer Sendeantenne (42), um aus den Untersuchungsimpulsen durch die Streuung der Untersuchungsimpulse an dem Brustbereich Streuimpulse zu erzeugen; Empfangen der Streuimpulse durch eine Empfangsantenne (44); Erzeugen eines Phasendifferenzsignals zwischen den Streuimpulsen und den Referenzimpulsen durch Mischen der Streuimpulse mit den Referenzimpulsen unter Verwendung eines Mischers (46); und Wandeln des Phasendifferenzsignals durch ein Signalverarbeitungsmodul (50), das mit einem Ausgangsanschluss des Mischers (46) verbunden ist, in ein digitales Signal, das die Herz-Lungen-Aktivität repräsentiert, durch Verarbeitung der Streuimpulse und der Referenzimpulse durch den Mischer (46); wobei die Referenzimpulse von einem ersten Eingangsanschluss (46a) des Mischers (46) empfangen werden und die Streuimpulse von einem zweiten Eingangsanschluss (46B) des Mischers empfangen werden, der mit der Empfangsantenne über ein erstes Hochfrequenzfilter (34B), das leitend mit einem Ausgangsanschluss der Empfangsantenne (44) verbunden ist, und über einen ersten rauscharmen Verstärker (32B) verbunden ist, der leitend mit dem Hochfrequenzfilter (34B) und dem Mischer (46) verbunden ist; eine Serie von hochfrequenten sinusförmigen Impulsen von dem Impuls-Generator (20) erzeugt wird, durch einen zweiten Verstärker (32A) verstärkt wird, der leitend mit einem Ausgangsanschluss des Impuls-Generators (20) verbunden ist, und durch ein zweites Hochfrequenzfilter (34A) gefiltert wird, und die wobei die Untersuchungsimpulse und die Referenzimpulse aus den hochfrequenten sinusförmigen Impulsen durch einen Verteiler (30) erzeugt werden, der leitend mit dem zweiten Hochfrequenzfilter (34A) verbunden ist, wobei das Frequenzband der Untersuchungsimpulse und der Referenzimpulse zwischen 2,7 GHz und 10 GHz liegt; und zum Wandeln des Phasendifferenzsignals in ein digitales Signal, das die Herz-Lungen-Aktivität repräsentiert, das Phasendifferenzsignal durch einen Niederfrequenzfilter (34C), das leitend mit dem Ausgangsanschluss des Mischers (46) verbunden ist, gefiltert wird, durch einen dritten Verstärker (34C) verstärkt wird, der leitend mit einem Ausgangsanschluss des Niederfrequenzfilters (34C) verbunden ist, und durch einen Bandpassfilter (56) gefiltert wird, der leitend mit dem Ausgangsanschluss des Verstärkers (34c) verbunden ist; wobei das Bandpassfilters (56) eine Basisliniendrift und ein hochfrequentes Rauschen entfernt, und die Herz-Lungen-Aktivitätssignale durch einen Analog-/Digital-Wandler (54) eines Mikroprozessors des Signalverarbeitungsmoduls (50) in digitale Signale gewandelt werden, wobei der Mikroprozessor (52) mit Hilfe eines mittels Software realisierten Bandpassfilters mit einer -3dB-Bandbreite von 0,1 Hz bis 0,5 Hz oder mit einer -3dB-Bandbreite von 0,7 Hz bis 2,5 Hz aus den digitalen Signalen Atmungs- oder Herzschlag-Signale extrahiert und daraus eine jeweilige Atemfrequenz oder Herzschlagfrequenz berechnet.

**7.** Verfahren nach Anspruch 6, wobei der Schritt des Berechnens einer Atemfrequenz oder Herzschlagfrequenz umfasst:

Einstellen eines Schwellenwerts; Prüfen um zu bestimmen, ob die Amplitude des digitalen Signals größer als der Schwellenwert ist, und um einen Signalspitzenwert der Herz-Lungen-Aktivität zu bestimmen, wenn das Prüfergebnis positiv ist; Berechnen von Zeitintervallen aus einer Mehrzahl von Signalspitzen der Herz-Lungen-Aktivität; und Mitteln der Zeitintervalle, um die Atemfrequenz oder Herzschlagfrequenz zu erzeugen.

**8.** Verfahren nach Anspruch 6 oder 7, wobei Atemsignale aus den digitalen Signalen extrahiert werden.

**9.** Verfahren nach einem der Ansprüche 6 bis 8, weiterhin umfassend die Schritte:

Prüfen, ob die berechnete Atemfrequenz oder Herzschlagfrequenz in einem vorbestimmten Bereich liegt; und Erzeugen eines Alarmsignals, wenn die Atemfrequenz oder Herzschlagfrequenz außerhalb des vorbestimmten Bereichs liegt.

**10.** Verfahren nach Anspruch 7, wobei der Schwellenwert entsprechend der Amplitude des digitalen Signals eingestellt wird.

**Revendications**

**1.** Un appareil sans contact pour la surveillance de signaux d'activités cardio-pulmonaires, comprenant:

un générateur d'impulsions en série (12) configuré pour générer une série d'impulsions de sondage et une série d'impulsions de référence;
une antenne d'émission (42) configurée pour transmettre les impulsions de sondage à une partie de la poitrine, dans lequel la partie de poitrine diffuse les impulsions de sondage pour générer une série d'impulsions diffuses ;
une antenne de réception (44) configurée pour recevoir les impulsions diffuses ;
un mélangeur (46) comprenant un premier port d'entrée (46A) configuré pour recevoir les impulsions de référence et un second port d'entrée (46B) configuré pour recevoir les impulsions diffuses et connecté à l'antenne de réception via un premier filtre haute fréquence (34B) électriquement connecté à un port de sortie de l'antenne de réception (44) et à un premier amplificateur faible bruit (32B) électriquement connecté au premier filtre haute fréquence (34B) et au mélangeur (46) ; et
un module de traitement de signal (50) connecté à un port de sortie du mélangeur (46) et configuré pour générer des signaux d'activité cardio-pulmonaires à la suite du traitement des impulsions diffuses et des impulsions de référence par le mélangeur (46) ;
dans lequel le générateur d'impulsions en série (12) comporte :

  un générateur d'impulsion (20) configuré pour générer une série d'impulsions sinusoïdales haute-fréquences ;
  un second amplificateur (32A) électriquement connecté à un port de sortie du générateur d'impulsions (20) ;
  un second filtre haute-fréquence (34A) électriquement connecté au second amplificateur (32A) ; et
  un séparateur (30) électriquement connecté au second filtre haute-fréquence (34A) et configuré pour générer les impulsions de sondage et les impulsions de référence à partir d'impulsions sinusoïdales haute-fréquences, dans lequel la bande de fréquence des impulsions de sondage et des impulsions de référence est entre 2.7 Ghz et 10 Ghz ; et
  dans lequel le module de traitement de signal (50) comporte :

    un filtre basse fréquence (34C) électriquement connecté à un port de sortie du mélangeur (46) ;
    un troisième amplificateur (34C) électriquement connecté à un port de sortie du filtre basse fréquence (34C) ;
    un filtre passe-bande (56) électriquement connecté au port de sortie du troisième amplificateur (34C) et configuré pour supprimer la dérive de ligne de base ainsi que le bruit haute fréquence ; et
    un microprocesseur ayant un convertisseur analogique/numérique (54) configuré pour convertir les signaux d'activité cardio-pulmonaire en signaux numériques ;
    dans lequel le microprocesseur (52) du module de traitement de signal (50) est configuré pour extraire les signaux de respiration ou cardiaques des signaux numériques au travers un filtre passe bande réalisé par logiciel avec une largeur de bande à -3dB depuis 0.1 Hz jusqu'à 0.5 Hz ou avec une largeur de bande à -3dB depuis 0.7 Hz à 2.5 Hz , respectivement, et pour en dériver une fréquence de respiration ou une fréquence cardiaque.

**2.** L'appareil sans contact de la revendication 1, dans lequel le générateur d'impulsions (20) comporte :

  un générateur de signal sinusoïdal (26) configuré pour générer un signal sinusoïdal continu ;
  un général de signal de commutation (22) configuré pour générer un signal de commutation ; et
  un dispositif de commutation (24) configuré pour la mise en fonctionnement en fonction du signal de commutation, de telle manière que le signal sinusoïdal continu peut travers le dispositif de commutation pour former les impulsions sinusoïdales haute-fréquences.

**3.** L'appareil sans contact de la revendication 2, dans lequel le générateur de signal de commutation (22) comporte :

  un générateur d'horloge (22A) configuré pour générer un signal d'horloge ; et
  un circuit de mise en forme d'onde (22B) configuré pour régler un intervalle de temps du signal d'horloge pour générer le signal de commutation.

**4.** L'appareil sans contact de l'une quelconque des revendications précédentes, dans lequel le module de traitement de signal (50) comporte en outre un générateur de signal d'alarme électriquement connecté au microprocesseur.

**5.** L'appareil sans contact de l'une quelconque des revendications précédentes, dans lequel le mélangeur est un mélangeur d'équilibrage (46).

**6.** Un procédé pour l'acquisition de signaux d'activité cardio-pulmonaire, comportant les étapes :

générer une série d'impulsions de sondage et une série d'impulsions de référence au moyen d'un générateur de série d'impulsions (12) ;

transmettre les impulsions de sondage à une partie de la poitrine au moyen d'une antenne de transmission (42) pour générer des impulsions diffuses à partir des impulsions de sondage au moyen de la diffusion des impulsions de sondage au niveau de la partie de la poitrine ;

recevoir les impulsions diffuses au moyen d'une antenne de réception (44) ;

générer un signal différentiel de phase entre les impulsions diffuses et les impulsions de référence en mélangeant les impulsions diffuses avec les impulsions de référence au moyen d'un mélangeur (46) ; et

convertir le signal différentiel de phase en un signal numérique représentatif de l'activité cardio-pulmonaire au moyen d'un module de traitement de signal (50) connecté à un port de sortie du mélangeur (46), au moyen d'un traitement des impulsions diffuses et des impulsions de référence par le mélangeur (46) ; dans lequel

les impulsions de référence sont reçues par un premier port d'entrée (46) du mélangeur (46) et les impulsions diffuses sont reçues par un second port d'entrée (46B) du mélangeur connecté à l'antenne de réception via un premier filtre haute fréquence (34B) électriquement connecté à un port de sortie de l'antenne de réception (44) et un premier amplificateur à faible bruit (32) électriquement connecté au premier filtre haute fréquence (34B) et au mélangeur (46) ;

une série d'impulsions sinusoïdales hautes fréquences étant générées par le générateur d'impulsions (20), amplifiées par un second amplificateur (32A) électriquement connecté à un port de sortie du générateur d'impulsions (20) et filtrés par un second filtre haute-fréquence (34A) et les impulsions de sondage et les impulsions de référence étant générées à partir d' impulsions sinusoïdales haute fréquence au moyen d'un séparateur (30) électriquement connecté au second filtre haute-fréquence (34A), dans lequel la bande de fréquence des impulsions de sondage et des impulsions de référence est entre 2.7 GHz et 10 Ghz ; et

pour la conversion du signal différentiel de phase en un signal numérique représentatif de l'activité cardio-pulmonaire, le signal différentiel de phase est filtré par un filtre basse fréquence (34C) électriquement connecté au port de sortie du mélangeur (46), amplifié par un troisième amplificateur (34C) électriquement connecté au port de sortie du filtre basse fréquence (34C) et filtré par un filtre passe-bande (56) électriquement connecté au port de sortie de l'amplificateur (34C) ; das lequel

le filtre passe-bande (56) supprime la dérive de ligne de base et le bruit haute-fréquence, et les signaux d'activité cardio-pulmonaire sont convertis en signaux numériques au moyen d'un convertisseur analogique/numérique (54) d'un microprocesseur du module de traitement de signal (50) ; dans lequel

le microprocesseur (52) extrait les signaux de respiration ou des signaux cardiaque à partir des signaux numériques via un filtre passe bande implémenté par voie logicielle, avec une largeur de bande à -3dB de 0.1 Hz à 0.5 Hz ou une largeur de bande à -3dB de 07 Hz à 2.5 Hz, respectivement, et en déduisant par calcul la fréquence respiratoire ou la fréquence cardiaque.

**7.** Le procédé de la revendication 6, dans lequel ladite étape de calcule d'une fréquence respiratoire ou d'une fréquence cardiaque comporte :

la configuration d'une valeur seuil ;

la vérification visant à déterminer si l'intensité du signal numérique est supérieure à la valeur de seuil et la détermination d'un pic de signal de l'activité cardio-pulmonaire lorsque le résultat de la vérification s'avère positif ;

le calcul d'intervalles temporels d'une pluralité de pics de signal de l'activité cardio-pulmonaire ; et

le calcul d'une moyenne des intervalles temporels pour générer la fréquence respiratoire ou la fréquence cardiaque.

**8.** Le procédé de la revendication 6 ou 7, dans lequel des signaux respiratoires sont extraits des signaux numériques.

**9.** Le procédé de l'une quelconque des revendications 6 à 8, comprenant en outre les étapes de:

vérification visant à déterminer si les fréquences respiratoire ou cardiaque se situent dans une gamme prédéterminée ; et

génération d'un signal d'alarme lorsque les fréquences respiratoire ou cardiaque se situent à l'extérieur de la gamme prédéterminée.

**10.** Le procédé de la revendication 7, dans lequel la valeur seuil est fixée en fonction de l'intensité du signal numérique.

10

44    46B    46    50

Balance mixer | Signal-processing module

46A

42    12

Pulse-series generator

FIG. 1

FIG. 2

**50**

**58**

| Alarm signal generator |
|---|

**52**

| Microprocessor |
|---|

**60**

| Memory |
|---|

**34C**

f = 0-15 Hz

Analog signal
(breath & heartbeat)

**32C**

**56**

| Band-pass filter |
|---|

| Analog/ Digital converter |
|---|

**54**

**62**

| Wireless transmission module |
|---|

# FIG. 3

FIG. 4

EP 1 935 333 B1

FIG. 5

FIG. 6

```
                    ┌─────────────┐
                    │    Start    │
                    └──────┬──────┘
                           │
                           ▼
              ┌────────────────────────────┐
              │   Acquiring the heartbeat   │
              │  signal by band-pass filter │
              └──────────────┬──────────────┘
                             │
                             ▼
         ┌─────────────────────────────────────────┐
         │  Setting the upper and lower threshold   │
         │  values-TBU ,TBL  according to the       │
         │  intensity of the measured digital signal│
         └─────────────────────┬───────────────────┘
                               │
                               ▼
                    ╱────────────────╲
                   ╱   Detecting the   ╲        Yes
                  ╱  peak of the heartbeat╲──────────────┐
                  ╲      signal?         ╱               │
                   ╲────────────────────╱                │
                           │ No                          │
                           ▼                             │
                    ╱────────────────╲                   │
                   ╱  No heartbeat     ╲      No          │
                  ╱  signal for 40      ╲────────────────┤
                  ╲     seconds?        ╱                 │
                   ╲────────────────────╱                 │
                           │ Yes                          │
                           ▼                              │
              ┌─────────────────────────────┐            │
              │   Generating the alarm signal│            │
              └──────────────┬──────────────┘            │
                             │                            │
                             ▼                            │
              ┌─────────────────────────────┐            │
              │   Resetting the heartbeat    │            │
              │         parameter            │            │
              └──────────────┬──────────────┘            │
                             │                            │
                             ▼                            │
                    ┌─────────────────────┐               │
                    │ Back to main program │◄─────────────┘
                    └─────────────────────┘
```

# FIG. 7

17

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4738264 A
- US 5964720 A
- WO 2005092190 A1
- WO 9420021 A
- GB 2349759 A
- WO 2007143535 A2